# EUROPÄISCHE PATENTSCHRIFT

(11) **EP 3 438 667 B1**
(45) Veröffentlichungstag und Bekanntmachung des Hinweises auf die Patenterteilung: **30.12.2020**
(21) Anmeldenummer: 17184389.9
(22) Anmeldetag: 02.08.2017
(51) Int. Cl.: G01N 33/68

(54) **BINDUNGSTEST ZUR DIAGNOSE EINER HEPARIN-INDUZIERTEN THROMBOZYTOPENIE**
BINDING TEST FOR THE DIAGNOSIS OF HEPARIN INDUCED THROMBOCYTOPENIA
ESSAI DE LIAISON POUR DIAGNOSTIQUER LA THROMBOCYTOPÉNIE INDUITE PAR L'HÉPARINE

(43) Veröffentlichungstag der Anmeldung: 06.02.2019
(73) Patentinhaber: Siemens Healthcare Diagnostics Products GmbH, 35041 Marburg (DE)
(72) Erfinder: Schwarz, Herbert, 35102 Lohra (DE); Althaus, Harald, 35083 Wetter (DE); Zander, Norbert, 35039 Marburg (DE)

(56) Entgegenhaltungen:
- A. CUKER ET AL: "Novel diagnostic assays for heparin-induced thrombocytopenia", BLOOD, Bd. 121, Nr. 18, 2. Mai 2013 (2013-05-02), Seiten 3727-3732, XP55223353, US ISSN: 0006-4971, DOI: 10.1182/blood-2013-01-479576
- T. BAKCHOUL ET AL: "Current insights into the laboratory diagnosis of HIT", INTERNATIONAL JOURNAL OF LABORATORY HEMATOLOGY, Bd. 36, Nr. 3, 18. April 2014 (2014-04-18) , Seiten 296-305, XP055404384, GB; US ISSN: 1751-5521, DOI: 10.1111/ijlh.12236

## Beschreibung

Die vorliegende Erfindung liegt auf dem Gebiet der in vitro-Diagnostik und betrifft einen leicht automatisierbaren Bindungstest zur Feststellung einer Heparin-induzierten Thrombozytopenie, bei dem mit FcyRIIa-Protein beschichtete Partikel eingesetzt werden.

Die Heparin-induzierte Thrombozytopenie (HIT) ist eine thrombotische Erkrankung, die im Rahmen einer Heparintherapie entstehen kann und lebensbedrohliche thromboembolische Komplikationen verursachen kann. Betroffene Patienten produzieren Antikörper, die einen Komplex aus Heparin und Plättchenfaktor 4 (PF4) binden, sogenannte anti-PF4/Heparin-Komplex-Antikörper. In vivo bindet der Antikörper-gebundene PF4/Heparin-Komplex an die Thrombozytenoberfläche und verursacht eine Aktivierung der Thrombozyten. Dadurch kommt es zu einer Abnahme der Thrombozytenzahl und einem erhöhten Risiko für Thromboembolien.

Zur HIT-Diagnose werden im Wesentlichen zwei Testprinzipien angewendet. Das erste Testprinzip beruht auf dem direkten Nachweis von anti-PF4/Heparin-Komplex-Antikörpern in einer Körperflüssigkeitsprobe eines Patienten. Dazu wird eine Probe mit PF4/Heparin-Komplex oder einem Komplex aus PF4 und einem anderen geeigneten Polyanion (wie z.B. Polyvinylsulfonat) in Kontakt gebracht, und die Bindung etwaiger in der Probe vorhandener anti-PF4/Heparin-Komplex-Antikörper mit herkömmlichen immunologischen Testverfahren (z.B. ELISA) nachgewiesen. Bakchoul, T. et al. ("Current insights into the laboratory diagnosis of HIT", International Journal of Laboratory Hematology 2014, 36 (3): 296-305) beschreiben einen kompetitiven Latex-Agglutinations-Assay, bei dem die Bindung eines zugegebenen anti-PF4/Heparin-Komplex-Antikörpers an mit PF4/Polyvinylsulfonat-beschichtete Partikel durch Antikörper aus der Probe gehemmt wird. Nachteilig ist jedoch, dass der Nachweis von anti-PF4/Heparin-Komplex-Antikörpern zwar sensitiv, aber nicht ausreichend spezifisch ist, d.h. der Nachweis der Antikörper ist nicht hinreichend für eine positive HIT-Diagnose, während ein negatives Ergebnis eine HIT angemessen sicher ausschließt. Daher wird die Bestätigung durch einen funktionellen Test basierend auf einem zweiten Testprinzip empfohlen.

Das zweite, funktionelle Testprinzip beruht auf dem Nachweis der Thrombozyten-aktivierenden Wirkung der anti-PF4/Heparin-Komplex-Antikörper. Bei diesem Testprinzip werden gewaschene Thrombozyten von einem oder mehreren normalen Spendern mit einer Plasma- oder Serumprobe eines Patienten und mit Heparin gemischt, und es wird die Thrombozytenaktivierung anhand bekannter Aktivierungsmarker, wie z.B. anhand der Menge von freigesetztem Serotonin (Serotoninfreisetzungstest), oder anhand der visuell erkennbaren Aggregationsreaktion der Thrombozyten (HIPA-Test) gemessen. Enthält eine Patientenprobe anti-PF4/Heparin-Komplex-Antikörper ist eine gegenüber einer normalen Probe (ohne derartige Antikörper) erhöhte Thrombozytenaktivierung feststellbar. Cuker, A. et al. ("Novel diagnostic assays for heparin-induced thrombocytopenia", Blood 2013, 121 (18): 3727-3732) beschreiben die Detektion zellaktivierender anti-PF4/Heparin-Komplex-Antikörper, wobei B-Lymphocyten, die mit einem Luciferase-Reportergen-gekoppelten FcγRIIa-Rezeptor transfiziert sind, mit der Probe in Kontakt gebracht werden und die Luciferase-Aktivität im Testansatz gemessen wird.

Die Thrombozyten-basierten, funktionellen Teste haben den Nachteil, dass die Thrombozyten in aufwändigen, manuellen Verfahren aufbereitet werden müssen und aufgrund ihrer mangelnden Haltbarkeit nur frisch eingesetzt werden können. Außerdem muss immer ein Gemisch von Thrombozyten mehrerer Spender verwendet werden, um biologische Varianzen zwischen individuellen Thrombozytenpräparationen auszugleichen, um so eine gewisse Standardisierung zu ermöglichen.

Der vorliegenden Erfindung liegt die Aufgabe zugrunde ein Verfahren zur Detektion von anti-PF4/Heparin-Komplex-Antikörpern in einer Körperflüssigkeitsprobe bereit zu stellen, bei dem die vorgenannten Nachteile, insbesondere die Verwendung von Thrombozyten, vermieden werden.

Es wurde gefunden, dass in einem Reaktionsgemisch, das durch Vermischen einer Probe mit Heparin oder PF4/Heparin-Komplex und mit einer nicht-zellulären partikulären Festphase und Bestimmung der Agglutination der nicht-zellulären partikulären Festphase das Vorhandensein von anti-PF4/Heparin-Komplex-Antikörpern in der Probe festgestellt werden kann, wenn die nicht-zelluläre partikuläre Festphase mit isoliertem FcγRIIa-Protein beschichtet ist.

Gegenstand der vorliegenden Erfindung ist also ein Verfahren zur Detektion von anti-PF4/Heparin-Komplex-Antikörpern in einer Körperflüssigkeitsprobe. Das Verfahren umfasst die Schritte:
i. Bereitstellen eines Reaktionsgemisches durch Vermischen der Probe
   - mit Heparin oder einem PF4-bindenden, unverzweigten Polysaccharid oder einem PF4-bindenden Polyanion, oder
   - mit PF4/Heparin-Komplex oder einem Komplex aus PF4 mit einem unverzweigten Polysaccharid oder einem Polyanion, und
   - mit einer nicht-zellulären partikulären Festphase;
ii. Messen der Agglutination der nicht-zellulären partikulären Festphase im Reaktionsgemisch;
iii. Vergleichen der so gemessenen Agglutination in dem Reaktionsgemisch mit einem vorbestimmten Referenzwert für die Agglutination in Reaktionsgemischen enthaltend Körperflüssigkeitsproben von Spendern, die bekanntermaßen keine anti-PF4/Heparin-Komplex-Antikörper enthalten; und
iv. Feststellen des Vorhandenseins von anti-PF4/Heparin-Komplex-Antikörpern in der Probe, wenn die in dem Reaktionsgemisch bestimmte Agglutination den Referenzwert überschreitet,
wobei die nicht-zelluläre partikuläre Festphase mit isoliertem FcγRIIa-Protein beschichtet ist.

Die Körperflüssigkeitsprobe stammt bevorzugterweise von einem Menschen. Bevorzugterweise handelt es sich um eine im Wesentlichen Thrombozyten-freie Körperflüssigkeitsprobe, insbesondere um Plasma oder Serum.

Die Körperflüssigkeitsprobe kann mit einem PF4-bindenden, unverzweigten Polysaccharid oder mit einem PF4-bindenden Polyanion vermischt werden. Es sind eine Vielzahl PF4-bindender Substanzen bekannt, die mit PF4 einen Komplex ausbilden, der von den zu detektierenden anti-PF4/Heparin-Komplex-Antikörpern gebunden wird. Geeignete unverzweigte Polysaccharide sind beispielsweise Heparin, unfraktioniertes Heparin (UFH), fraktioniertes Heparin (LMWH), Dextransulfat und Fucoidan. Geeignete Polyanionen sind beispielsweise Polyvinylsulfat, Polyvinylsulfonat, Polyvinylphosphat, Polyvinylphosphonat, Polystyrensulfat und Polystyrensulfonat.

Alternativ kann die Körperflüssigkeitsprobe mit PF4/Heparin-Komplex oder mit einem Komplex aus PF4 mit einem unverzweigten Polysaccharid oder einem Polyanion vermischt werden.

Weiterhin wird die Probe mit einer nicht-zellulären partikulären Festphase vermischt, die mit isoliertem FcγRIIa-Protein beschichtet ist.

Unter dem Begriff "partikuläre Festphase" sind im Sinne dieser Erfindung nicht-zelluläre Teilchen zu verstehen, die einen ungefähren Durchmesser von wenigstens 20 nm und nicht mehr als 20 µm aufweisen, üblicherweise zwischen 200 nm und 350 nm, bevorzugt zwischen 250 und 320 nm, besonders bevorzugt zwischen 270 und 290 nm, ganz besonders bevorzugt 280 nm. Die Mikropartikel können regelmäßig oder unregelmäßig geformt sein. Sie können Kugeln, Spheroide, Kugeln mit mehr oder weniger großen Kavitäten oder Poren darstellen. Die Mikropartikel können aus organischem, aus anorganischem Material oder aus einer Mischung oder einer Kombination von beiden bestehen. Sie können aus einem porösen oder nicht porösen, einem schwellfähigen oder nicht schwellfähigen Material bestehen. Prinzipiell können die Mikropartikel jegliche Dichte haben, bevorzugt sind jedoch Partikel mit einer Dichte, die der Dichte des Wassers nahe kommt wie etwa 0,7 bis etwa 1,5 g/ml. Die bevorzugten Mikropartikel sind in wässrigen Lösungen suspendierbar und möglichst lange suspensionsstabil. Sie mögen durchsichtig, teilweise durchsichtig oder undurchsichtig sein. Die Mikropartikel können aus mehreren Schichten bestehen wie beispielsweise die sogenannten "core-and-shell" Partikel mit einem Kern und einer oder mehreren umhüllenden Schichten. Der Begriff Mikropartikel umfasst beispielsweise Farbstoffkristalle, Metallsolen, Silica-Partikel, Glaspartikel und Magnetpartikel. Bevorzugte Mikropartikel sind in wässrigen Lösungen suspendierbare und aus wasserunlöslichem Polymermaterial bestehende Partikel, insbesondere aus substituierten Polyethylenen. Ganz besonders bevorzugt sind Latexpartikel z.B. aus Polystyrol, Acrylsäurepolymeren, Methacrylsäurepolymeren, Acrylnitril-Polymeren, AcrylnitrilButadien-Styrol, Polyvinylacetat-Acrylat, Polyvinylpyridin, Vinylchlorid-Acrylat. Von besonderem Interesse sind Latexpartikel mit reaktiven Gruppen an ihrer Oberfläche wie beispielsweise Carboxyl-, Amino- oder Aldehydgruppen, die eine kovalente Bindung von isoliertem FcγRIIa-Protein an die Latexpartikel erlauben. Humane, tierische, pflanzliche oder pilzliche Zellen oder Bakterien sind im Sinne dieser Erfindung explizit nicht von dem Begriff "partikuläre Festphase" umfasst.

Unter dem Begriff "isoliertes FcγRIIa-Protein" ist ein rekombinant oder synthetisch hergestelltes FcγRIIa-Protein oder ein natives, das heißt aus natürlichen Quellen, z.B. aus humanen Leukozyten, gereinigtes FcγRIIa-Protein zu verstehen. Zur Herstellung von rekombinantem FcγRIIa-Protein eignen sich bekannte prokaryontische oder eukaryontische Expressionssysteme, wie z.B. die Expression in Bakterien (z.B. E. coli), in Hefen (z.B. Saccharomyces cerevisiae, Pichia pastoris), in pflanzlichen, tierischen oder humanen Zellkulturen. Zur Herstellung von synthetischem FcyRIIa-Protein eignen sich bekannte Techniken zur in vitro Proteinsynthese, wie z. B. Festphasensynthesen (z.B. Merrifield-Synthese). Bevorzugterweise handelt es sich bei dem in dem erfindungsgemäßen Verfahren verwendeten FcγRIIa-Protein um rekombinant hergestelltes FcγRIIa-Protein, das in einer Kultur humaner Zellen, bevorzugt in einer Kultur humaner embryonaler Nierenzellen (HEK-Zellen), hergestellt wurde.

Das FcγRIIa-Protein (synonym: CD32a-Protein) ist vorzugsweise humanes FcγRIIa-Protein. Der Begriff "FcγRIIa-Protein" umfasst nicht nur das vollständige, sondern auch Fragmente des vollständigen FcγRIIa-Proteins, die fähig sind an Immunkomplexe aus PF4/Heparin-Komplex und daran gebundene anti-PF4/Heparin-Komplex-Antikörper zu binden. Der Begriff "FcγRIIa-Protein" umfasst ferner nicht nur das wildtypische FcγRIIa-Protein oder Fragmente davon, sondern auch FcγRIIa-Proteine mit einer oder mehr Aminosäuresubstitutionen, die fähig sind an PF4/Heparin-Komplex zu binden, wie z.B. eine bekannte Substitution an Position 131 des humanen FcγRIIa-Proteins. Das FcγRIIa-Protein bzw. ein FcγRIIa-Proteinfragment kann am N-Terminus mit einer heterologen Signalsequenz fusioniert sein, d.h. mit einem Polypeptid, das üblicherweise nicht in dem humanen FcγRIIa-Protein vorhanden ist, die aber in dem gewählten Expressionssystem die Expression und/oder Sekretion des rekombinant exprimierten FcγRIIa-Proteins positiv beeinflusst. Weiterhin kann das FcγRIIa-Protein bzw. ein FcγRIIa-Proteinfragment am C-Terminus mit einem oder mehreren Affinitäts-Tags fusioniert sein, die die Bindung des z.B. rekombinant exprimierten Proteins an einen Affinitätsträger ermöglichen, wodurch z.B. die Reinigung von rekombinant exprimiertem FcγRIIa-Protein ermöglicht wird. Bevorzugt sind kleine Affinitäts-Tags mit einer Länge von nicht mehr als 12 Aminosäuren. Besonders bevorzugt sind Affinitäts-Tags aus der Gruppe His-Tag, Flag-Tag, Arg-Tag, c-Myc-Tag und Strep-Tag. Geeignete Affinitätsträger, die mit hoher Affinität an ein Affinitäts-Tag binden, sind z.B. spezifische Antikörper, immobilisierte Kationen (z. B. Ni²⁺ mit Affinität für His-Tags) oder andere Typen von Bindungspartnern (z.B. Streptavidin mit Affinität für Strep-Tags).

Sind in der Körperflüssigkeitsprobe anti-PF4/Heparin-Komplex-Antikörper enthalten, binden diese in dem Reaktionsgemisch an PF4/Heparin-Komplexe. Die formierten Immunkomplexe werden vom FcγRIIa-Protein gebunden und bewirken eine Agglutination der partikulären Festphase im Reaktionsgemisch.

Die Messung der Agglutination der partikulären Festphase im Reaktionsgemisch kann photometrisch erfolgen, beispielsweise turbidimetrisch oder nephelometrisch. Bindungsteste beruhend auf dem Prinzip der partikelverstärkten Lichtstreuung sind seit etwa 1920 bekannt (zur Übersicht siehe Newman, D.J. et al., Particle enhanced light scattering immunoassay. Ann Clin Biochem 1992; 29: 22-42). Bevorzugterweise werden in diesem Zusammenhang Polystyrolpartikel mit einem Durchmesser von 0,1 bis 0,5 µm, besonders bevorzugt mit einem Durchmesser von 0,15 bis 0,35 µm verwendet. Bevorzugt werden Polystyrolpartikel mit Amin-, Carboxyl- oder Aldehydfunktionen verwendet. Weiterhin bevorzugt werden Schale/Kern-Partikel verwendet. Die Synthese der Partikel und die kovalente Kopplung von Liganden ist z.B. in Peula, J.M. et al., Covalent coupling of antibodies to aldehyde groups on polymer carriers. Journal of Materials Science: Materials in Medicine 1995; 6: 779-785 beschrieben.

Alternativ kann die Messung der Agglutination der partikulären Festphase im Reaktionsgemisch durch die Messung eines Signals erfolgen, das von einem signalbildenden System erzeugt wird, wenn eine erste und eine zweite Komponente des signalbildenden Systems in räumliche Nähe zueinander gebracht werden. In diesem Zusammenhang ist eine erste Fraktion der partikulären Festphase mit einer ersten Komponente eines signalbildenden Systems assoziiert, und eine zweite Fraktion der partikulären Festphase ist mit einer zweiten Komponente des signalbildenden Systems assoziiert, wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden, und die Agglutination der partikulären Festphase im Reaktionsgemisch anhand des entstandenen Signals gemessen wird.

In dieser Ausführungsform des erfindungsgemäßen Verfahrens umfasst das signalbildende System mindestens eine erste und eine zweite Komponente, welche so zusammenwirken, dass ein detektierbares Signal entsteht, wenn sie in räumliche Nähe zueinander gebracht werden und dadurch miteinander in Wechselwirkung treten können. Unter einer Wechselwirkung zwischen den Komponenten ist insbesondere ein Energietransfer - also die direkte Übertragung von Energie zwischen den Komponenten, z.B. durch Licht- oder Elektronenstrahlung sowie über reaktive chemische Moleküle, wie z.B. kurzlebigen Singulett-Sauerstoff - zu verstehen. Der Energietransfer kann von einer auf eine andere Komponente erfolgen, möglich ist aber auch eine Kaskade verschiedener Substanzen über die der Energietransfer läuft. Zum Beispiel kann es sich bei den Komponenten um ein Paar aus einem Energiespender und einem Energieempfänger handeln, wie beispielsweise Photosensitizer und chemilumineszierendes Agens (EP-A2-0515194, LOCI® Technologie) oder Photosensitizer und Fluorophor (WO 95/06877) oder radioaktives Iod<125> und Fluorophor (Udenfriend et al. (1985) Proc. Natl. Acad. Sci. 82: 8672 - 8676) oder Fluorophor und Fluoreszenz-Quencher (US 3,996,345). Besonders bevorzugt ist die erste Komponente des signalbildenden Systems ein chemilumineszierendes Agens und die zweite Komponente des signalbildenden Systems ein Photosensitizer oder umgekehrt, und es wird die Chemilumineszenz im Reaktionsgemisch gemessen.

Nachdem die Agglutination in dem Reaktionsgemisch gemessen wurde (z.B. durch Bestimmung der maximalen Absorptionsänderung des Reaktionsgemisches), wird die so gemessene Agglutination mit einem vorbestimmten Referenzwert verglichen. Als Referenzwert eignet sich die Agglutination, die mit demselben Verfahren in Reaktionsgemischen gemessen wird (oder vorab gemessen wurde) enthaltend Körperflüssigkeitsproben von Spendern, die bekanntermaßen keine anti-PF4/Heparin-Komplex-Antikörper enthalten. Üblicherweise wird zur Bestimmung eines Referenzwertes in einer Vielzahl von Proben von gesunden Spendern, die bekanntermaßen keine anti-PF4/Heparin-Komplex-Antikörper aufweisen, die Agglutination gemessen und dann mit der Agglutination einer Vielzahl von Proben von an HIT erkrankten Spendern, die anti-PF4/Heparin-Komplex-Antikörper aufweisen, verglichen. Ein Referenzwert kann beispielsweise dann ein Grenzwert sein, der die Differenzierung von Proben mit und Proben ohne anti-PF4/Heparin-Komplex-Antikörpern ermöglicht. Überschreitet die in einem Reaktionsgemisch gemessene Agglutination den Referenzwert, erlaubt dies das Feststellen des Vorhandenseins von anti-PF4/Heparin-Komplex-Antikörpern in der Probe. Wenn die in dem Reaktionsgemisch gemessene Agglutination den Referenzwert hingegen unterschreitet, erlaubt dies das Feststellen des Fehlens von anti-PF4/Heparin-Komplex-Antikörpern in der Probe.

Ein weiterer Gegenstand der vorliegenden Erfindung ist ein Verfahren zur Diagnose einer Heparin-induzierten Thrombozytopenie, wobei mit einem erfindungsgemäßen Verfahren das Vorhandensein von anti-PF4/Heparin-Komplex-Antikörpern in einer Körperflüssigkeitsprobe eines Patienten detektiert wird.

Noch ein weiterer Gegenstand der vorliegenden Erfindung ist ein Testkit zur Durchführung eines erfindungsgemäßen Verfahrens. Das Testkit enhält mindestens folgende Komponenten:
a. ein erstes Reagenz enthaltend Heparin, ein PF4-bindendes, unverzweigtes Polysaccharid oder ein PF4-bindendes Polyanion oder PF4/Heparin-Komplex oder einen Komplex aus PF4 mit einem unverzweigten Polysaccharid oder einem Polyanion; und
b. ein zweites Reagenz enthaltend eine nicht-zelluläre partikuläre Festphase, die mit isoliertem FcγRIIa-Protein beschichtet ist.

Das erste Reagenz kann entweder
- ein PF4-bindendes, unverzweigtes Polysaccharid, bevorzugt aus der Gruppe Heparin, unfraktioniertes Heparin, fraktioniertes Heparin, Dextransulfat und Fucoidan; oder
- ein PF4-bindendes Polyanion, bevorzugt aus der Gruppe Polyvinylsulfat, Polyvinylsulfonat, Polyvinylphosphat, Polyvinylphosphonat, Polystyrensulfat und Polystyrensulfonat; oder
- PF4/Heparin-Komplex; oder
- einen Komplex aus PF4 mit einem unverzweigten Polysaccharid oder einem Polyanion
enthalten.

Das erste und das zweite Reagenz sind zur Bereitstellung des Reaktionsgemisches mit der Körperflüssigkeitsprobe vorgesehen.

In einem bevorzugten Testkit besteht die in dem zweiten Reagenz enthaltene nicht-zelluläre partikuläre Festphase aus Latexpartikeln. Ein solches Testkit eignet sich für die Messung der Agglutination mittels photometrischer Methoden.

Eine andere Ausführungsform des Testkits enthält neben dem ersten und zweiten Reagenz ferner ein drittes Reagenz, das ebenfalls eine nicht-zelluläre partikuläre Festphase enthält, die mit isoliertem FcγRIIa-Protein beschichtet ist. Die nicht-zelluläre partikuläre Festphase des zweiten Reagenzes ist in diesem Zusammenhang mit einer ersten Komponente eines signalbildenden Systems assoziiert und die nicht-zelluläre partikuläre Festphase des dritten Reagenzes ist mit einer zweiten Komponente des signalbildenden Systems assoziiert, wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden. Vorzugsweise ist die erste Komponente des signalbildenden

Systems ein chemilumineszierendes Agens, und die zweite Komponente des signalbildenden Systems ist ein Photosensitizer oder umgekehrt. Ein solches Testkit eignet sich für die Messung der Agglutination mittels Chemilumineszenzmessung.

In noch einer anderen Ausführungsform des Testkits ist das in dem ersten Reagenz enthaltene Heparin, PF4-bindende, unverzweigte Polysaccharid, PF4-bindende Polyanion oder der in dem ersten Reagenz enthaltene PF4/Heparin-Komplex oder der Komplex aus PF4 mit einem unverzweigten Polysaccharid oder einem Polyanion an eine weitere nicht-zelluläre partikuläre Festphase, vorzugsweise an Latexpartikel, gekoppelt.

Die Reagenzien eines erfindungsgemäßen Testkits können in flüssiger oder lyophilisierter Form bereitgestellt werden. Für den Fall, dass einige oder alle Reagenzien des Testkits als Lyophilisate vorliegen, kann das Testkit zusätzlich die zur Lösung der Lyophilisate erforderlichen Lösemittel enthalten, wie z.B. destilliertes Wasser oder geeignete Puffer.

Die folgenden Beispiele dienen der Veranschaulichung der vorliegenden Erfindung und sind nicht als Einschränkung zu verstehen.

### BEISPIELE

### Beispiel 1: Homogener Bindungstest zur Detektion von anti-PF4/Heparin-Komplex-Antikörpern

Reagenz 1 wurde durch Mischen von aus humanem Thrombozytenkonzentrat isoliertem PF4-Protein mit unfraktioniertem Heparin (UFH) in einem Verhältnis von 10 µg PF4-Protein : 0,2 U/mL (UFH) in einer Pufferlösung hergestellt. Alternativ wurde ein Reagenz 1 verwendet, das lediglich unfraktioniertes Heparin (UFH) (0,2 U/mL) enthielt.

Zur Herstellung des Reagenzes 2 wurden etwa 1 mg humanes FcγRIIa-Protein mit 1 mL Polystyrol-Latex-Partikeln (50 mg/mL, Teilchendurchmesser 0,2-0,3 µm) in einer Pufferlösung vermischt und inkubiert. Nach mehrmaligem Waschen der Partikel wurden die Partikel anschließend in 50 mL einer Pufferlösung resuspendiert.

20 µL einer humanen Serumprobe wurden mit 20 µL des Reagenzes 1 enthaltend PF4/Heparin-Komplex und 80 µL des Reagenzes 2 enthaltend mit FcyRIIa-Protein beschichtete Latex-Partikel zu einem Reaktionsgemisch vermischt und bei 37 °C inkubiert.

Die optische Dichte (OD) des Reaktionsgemisches wurde über einen Zeitraum von 6 Minuten bei einer Wellenlänge von 570 nm in einem BCS XP Analysegerät (Siemens Healthcare Diagnostics Products GmbH) gemessen, und als Messergebnis wurde die mittlere Änderung der optischen Dichte pro Minute ermittelt (Delta OD/min).

Mit dem erfindungsgemäßen Agglutinationstest wurden Serumproben von 5 HIT-Patienten gemessen, bei denen anhand klinischer Kriterien (4T-Score, zum Teil mit thrombotischem Ereignis) eine HIT diagnostiziert und das Vorliegen von anti-PF4/Heparin-Komplex-Antikörpern mit zwei unabhängigen, kommerziell erhältlichen Immunoassays (HemosIL® AcuStar HIT-Ab(PF4-H), Instrumentation Laboratories und Asserachrom® HPIA-IgG, Diagnostica Stago) festgestellt worden war.

Ferner wurden Serumproben von 4 gesunden Spendern (die keine klinischen HIT-Kriterien und auch keine anti-PF4/Heparin-Komplex-Antikörper aufwiesen) und ein normaler Plasmapool (aus etwa 20 Plasmen gesunder Spender, "FNP") gemessen.

Als Negativkontrolle wurde Puffer eingesetzt.

In Tabelle 1 sind die Testergebnisse zusammengefasst. Es wird nach Testergebnissen unterschieden, bei denen entweder Reagenz 1 enthaltend PF4/Heparin-Komplex (PF4/Heparin) oder Reagenz 1 enthaltend nur Heparin (Heparin) verwendet wurde.

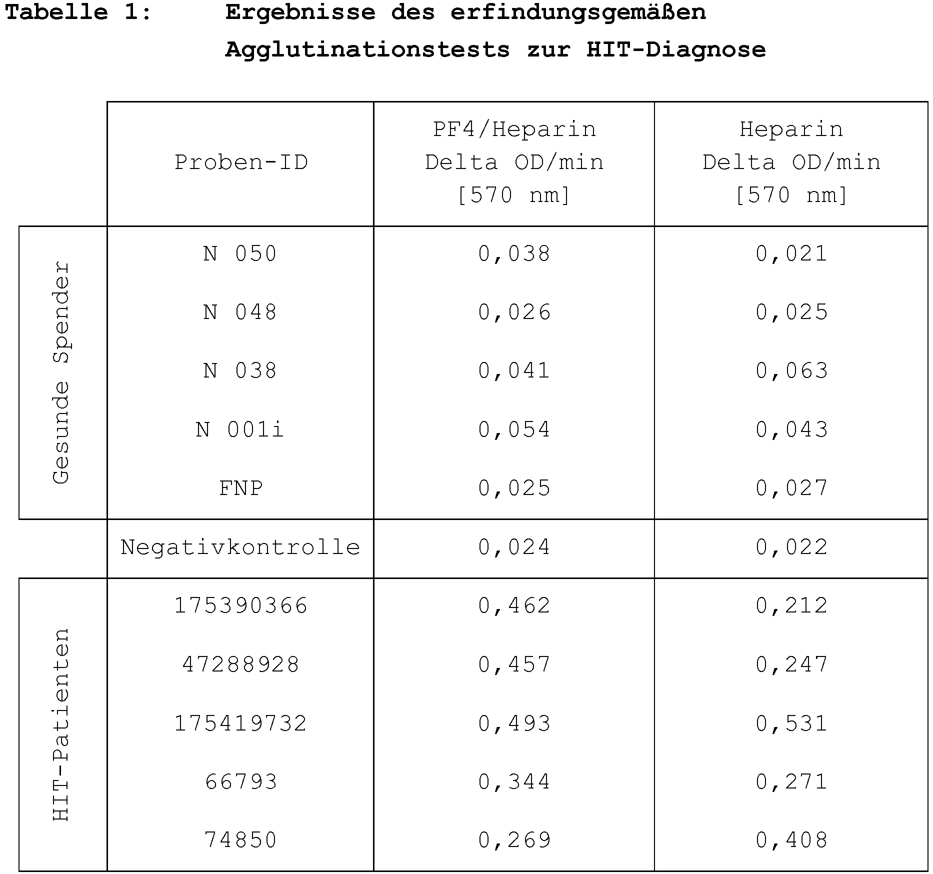

Es zeigt sich, dass die Ergebnisse beider erfindungsgemäßen Agglutinationstests eine Differenzierung von gesunden, HITnegativen Blutspendern und HIT-positiven Patienten ermöglichen. In allen Reaktionsgemischen enthaltend HIT-Patientenproben ist eine gegenüber gesunden Spendern signifikant erhöhte Agglutination messbar. Als Grenzwert (Cut-Off) für die Differenzierung von Proben, die anti-PF4/Heparin-Komplex-Antikörper enthalten, von solchen die keine enthalten, könnte in den beiden vorliegenden Testsystemen ein Delta OD/min [570 nm] von 0,1 gewählt werden.

Für eine statistisch genauere Festlegung des Cut-Off-Wertes ist jedoch eine deutliche höhere Anzahl von Probenmessungen erforderlich.

## Patentansprüche

1. Verfahren zur Detektion von anti-PF4/Heparin-Komplex-Antikörpern in einer Körperflüssigkeitsprobe, das Verfahren umfassend die Schritte:
i. Bereitstellen eines Reaktionsgemisches durch Vermischen der Probe
• mit Heparin oder einem PF4-bindenden, unverzweigten Polysaccharid oder einem PF4-bindenden Polyanion, oder
• mit PF4/Heparin-Komplex oder einem Komplex aus PF4 mit einem unverzweigten Polysaccharid oder einem Polyanion, und
• mit einer nicht-zellulären partikulären Festphase; und
ii. Messen der Agglutination der nicht-zellulären partikulären Festphase im Reaktionsgemisch;
iii. Vergleichen der so gemessenen Agglutination in dem Reaktionsgemisch mit einem vorbestimmten Referenzwert für die Agglutination in Reaktionsgemischen enthaltend Körperflüssigkeitsproben von Spendern, die bekanntermaßen keine anti-PF4/Heparin-Komplex-Antikörper enthalten; und
iv. Feststellen des Vorhandenseins von anti-PF4/Heparin-Komplex-Antikörpern in der Probe, wenn die in dem Reaktionsgemisch bestimmte Agglutination den Referenzwert überschreitet,
**dadurch gekennzeichnet, dass**
die nicht-zelluläre partikuläre Festphase mit isoliertem FcγRIIa-Protein beschichtet ist.

2. Verfahren gemäß Anspruch 1, wobei die Agglutination der nicht-zellulären partikulären Festphase im Reaktionsgemisch photometrisch gemessen wird.

3. Verfahren gemäß Anspruch 1, wobei eine erste Fraktion der nicht-zellulären partikulären Festphase mit einer ersten Komponente eines signalbildenden Systems assoziiert ist und eine zweite Fraktion der nicht-zellulären partikulären Festphase mit einer zweiten Komponente des signalbildenden Systems assoziiert ist, und wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden und die Agglutination der nicht-zellulären partikulären Festphase im Reaktionsgemisch anhand des entstandenen Signals gemessen wird.

4. Verfahren gemäß Anspruch 3, wobei die erste Komponente des signalbildenden System ein chemilumineszierendes Agens ist und die zweite Komponente des signalbildenden Systems ein Photosensitizer ist oder umgekehrt und wobei die Chemilumineszenz im Reaktionsgemisch gemessen wird.

5. Verfahren zur Diagnose einer Heparin-induzierten Thrombozytopenie, wobei mit einem Verfahren gemäß einem der Ansprüche 1 bis 4 das Vorhandensein von anti-PF4/Heparin-Komplex-Antikörpern in einer Körperflüssigkeitsprobe eines Patienten detektiert wird.

6. Testkit zur Durchführung eines Verfahrens gemäß einem der Ansprüche 1 bis 5, welches folgende Komponenten enthält:
a. ein erstes Reagenz enthaltend Heparin, ein PF4-bindendes, unverzweigtes Polysaccharid oder ein PF4-bindendes Polyanion oder PF4/Heparin-Komplex oder einen Komplex aus PF4 mit einem unverzweigten Polysaccharid oder einem Polyanion; und
b. ein zweites Reagenz enthaltend eine nicht-zelluläre partikuläre Festphase, die mit isoliertem FcγRIIa-Protein beschichtet ist.

7. Testkit gemäß Anspruch 6, welches ferner folgende Komponente enthält:
c. ein drittes Reagenz enthaltend eine nicht-zelluläre partikuläre Festphase, die mit isoliertem FcγRIIa-Protein beschichtet ist,
wobei die nicht-zelluläre partikuläre Festphase des zweiten Reagenzes mit einer ersten Komponente eines signalbildenden Systems assoziiert ist und die nicht-zelluläre partikuläre Festphase des dritten Reagenzes mit einer zweiten Komponente des signalbildenden Systems assoziiert ist, und wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden.

8. Testkit gemäß Anspruch 7, wobei die erste Komponente des signalbildenden System ein chemilumineszierendes Agens ist und die zweite Komponente des signalbildenden Systems ein Photosensitizer ist oder umgekehrt.

9. Testkit gemäß einem der Ansprüche 6 bis 8, wobei die nicht-zelluläre partikuläre Festphase Latexpartikel sind.

10. Testkit gemäß einem der Ansprüche 6 bis 9, wobei das in dem ersten Reagenz enthaltene Heparin, PF4-bindende, unverzweigte Polysaccharid, PF4-bindende Polyanion oder der in dem ersten Reagenz enthaltene PF4/Heparin-Komplex oder der Komplex aus PF4 mit einem unverzweigten Polysaccharid oder einem Polyanion an eine weitere nicht-zelluläre partikuläre Festphase, vorzugsweise an Latexpartikel, gekoppelt ist.

## Claims

1. Method for detecting anti-PF4/heparin complex antibodies in a body-fluid sample, the method comprising the steps:
i. providing a reaction mixture by mixing the sample
• with heparin or a PF4-binding, unbranched polysaccharide or a PF4-binding polyanion, or
• with PF4/heparin complex or a complex composed of PF4 with an unbranched polysaccharide or a polyanion, and
• with a non-cellular particulate solid phase; and
ii. measuring the agglutination of the non-cellular particulate solid phase in the reaction mixture;
iii. comparing the thus measured agglutination in the reaction mixture with a predetermined reference value for the agglutination in reaction mixtures containing body-fluid samples from donors known to contain no anti-PF4/heparin complex antibodies; and
iv. establishing the presence of anti-PF4/heparin complex antibodies in the sample when the agglutination determined in the reaction mixture exceeds the reference value,
**characterized in that**
the non-cellular particulate solid phase has been coated with isolated FcγRIIa protein.

2. Method according to Claim 1, wherein the agglutination of the non-cellular particulate solid phase in the reaction mixture is measured photometrically.

3. Method according to Claim 1, wherein a first fraction of the non-cellular particulate solid phase has been associated with a first component of a signal-forming system and a second fraction of the non-cellular particulate solid phase has been associated with a second component of the signal-forming system, and wherein the first and second component of the signal-forming system interact such that a detectable signal is formed when the first and the second component of the signal-forming system are brought into close proximity to one another, and the agglutination of the non-cellular particulate solid phase in the reaction mixture is measured on the basis of the signal which is formed.

4. Method according to Claim 3, wherein the first component of the signal-forming system is a chemiluminescent agent and the second component of the signal-forming system is a photosensitizer or vice versa, and wherein the chemiluminescence in the reaction mixture is measured.

5. Method for diagnosing a heparin-induced thrombocytopenia, wherein a method according to any of Claims 1 to 4 is used to detect the presence of anti-PF4/heparin complex antibodies in a body-fluid sample from a patient.

6. Assay kit for carrying out a method according to any of Claims 1 to 5, containing the following components:
a. a first reagent containing heparin, a PF4-binding, unbranched polysaccharide or a PF4-binding polyanion or PF4/heparin complex or a complex composed of PF4 with an unbranched polysaccharide or a polyanion; and
b. a second reagent containing a non-cellular particulate solid phase which has been coated with isolated FcγRIIa protein.

7. Assay kit according to Claim 6, further containing the following component:
c. a third reagent containing a non-cellular particulate solid phase which has been coated with isolated FcγRIIa protein,
wherein the non-cellular particulate solid phase of the second reagent has been associated with a first component of a signal-forming system and the non-cellular particulate solid phase of the third reagent has been associated with a second component of the signal-forming system, and wherein the first and second component of the signal-forming system interact such that a detectable signal is formed when the first and the second component of the signal-forming system are brought into close proximity to one another.

8. Assay kit according to Claim 7, wherein the first component of the signal-forming system is a chemiluminescent agent and the second component of the signal-forming system is a photosensitizer or vice versa.

9. Assay kit according to any of Claims 6 to 8, wherein the non-cellular particulate solid phase are latex particles.

10. Assay kit according to any of Claims 6 to 9, wherein the heparin, PF4-binding, unbranched polysaccharide or PF4-binding polyanion that is present in the first reagent or the PF4/heparin complex that is present in the first reagent or the complex composed of PF4 with an unbranched polysaccharide or a polyanion has been coupled to a further non-cellular particulate solid phase, preferably to latex particles.

## Revendications

1. Procédé de détection d'anticorps de complexe d'anti-PF4/héparine dans un échantillon de liquide corporel, le procédé comprenant les stades :
i. on se procure un mélange de réaction en mélangeant l'échantillon
• à de l'héparine ou à un polysaccharide non ramifié fixant PF4 ou à un polyanion fixant PF4, ou
• à un complexe de PF4/héparine ou à un complexe composé de PF4 avec un polysaccharide non ramifié ou un polyanion ; et
• à une phase solide particulaire non cellulaire ; et
ii on mesure l'agglutination de la phase solide particulaire non cellulaire dans le mélange de réaction ;
iii on compare l'agglutination ainsi mesurée dans le mélange de réaction à une valeur de référence déterminée à l'avance de l'agglutination dans des mélanges de réaction contenant des échantillons de liquide corporel de donneurs, qui, d'une manière connue, ne contiennent pas d'anticorps de complexe d'anti-PF4/héparine ; et
iv on constate la présente d'anticorps de complexe d'anti-PF4/héparine dans l'échantillon, si l'agglutination déterminée dans le mélange de réaction dépasse la valeur de référence,
**caractérisé en ce que**
la phase solide particulaire non cellulaire est revêtue de la protéine FcγRIIa isolée.

2. Procédé suivant la revendication 1, dans lequel on mesure par photométrie l'agglutination de la phase solide particulaire non cellulaire dans le mélange de réaction.

3. Procédé suivant la revendication 1, dans lequel une première fraction de la phase solide particulaire non cellulaire est associée à un premier constituant d'un système formant un signal et une deuxième fraction de la phase solide particulaire non cellulaire est associée à un deuxième constituant du système formant un signal et dans lequel le premier et le deuxième constituants du système formant un signal coopèrent, de manière à créer un signal détectable, si le premier et le deuxième constituants du système formant un signal sont mis à proximité l'un de l'autre dans l'espace et on mesure, à l'aide du signal créé, l'agglutination de la phase solide particulaire non cellulaire dans le mélange de réaction.

4. Procédé suivant la revendication 3, dans lequel le premier constituant du système formant un signal est un agent chemioluminescent et le deuxième constituant du système formant un signal est un photosensibilisateur ou inversement, et dans lequel on mesure la chemioluminescence du mélange réactionnel.

5. Procédé de diagnostic d'une trombocytopénie induite par l'héparine, dans lequel on détecte, par un procédé suivant l'une des revendications 1 à 4, la présence d'anticorps de complexe d'anti-PF4/héparine dans un échantillon de liquide corporel d'un patient.

6. Trousse de test pour effectuer un procédé suivant l'une des revendications 1 à 5, qui contient les constituants suivants :
a. un premier réactif contenant de l'héparine, un polysaccharide non ramifié fixant PF4 ou un polyanion fixant PF4 ou un complexe de PF4/héparine ou un complexe composé de PF4 avec un polysaccaride non ramifié ou un polyanion ; et
b. un deuxième réactif contenant une phase solide particulaire non cellulaire, qui est revêtue de la protéine FcγRIIa isolée.

7. Trousse de test suivant la revendication 6, qui contient, en outre, les constituants suivants :
c. un troisième réactif contenant une phase solide particulaire non cellulaire, qui est revêtue de la protéine FcγRIIa isolée,
dans laquelle la phase solide particulaire non cellulaire du deuxième réactif est associée à un premier constituant d'un système formant un signal et la phase solide particulaire non cellulaire du troisième réactif est associée à un deuxième constituant du système formant un signal, et dans laquelle le premier et le deuxième constituants du système formant un signal coopèrent, de manière à créer un signal détectable, si le premier et le deuxième constituants du système formant un signal sont mis à proximité l'une de l'autre dans l'espace.

8. Trousse de test suivant la revendication 7, dans laquelle le premier constituant du système formant un signal est un agent chemioluminescent et le deuxième constituant du système formant un signal est un photosensibilisateur, ou inversement.

9. Trousse de test suivant l'une des revendications 6 à 8, dans laquelle les phases solides particulaires non cellulaires sont des particules de latex.

10. Trousse de test suivant l'une des revendications 6 à 9, dans laquelle l'héparine contenue dans le premier réactif, un polysaccharide non ramifié fixant PF4, un polyanion fixant PF4 ou le complexe de PF4/héparine contenu dans le premier réactif ou le complexe composé de PF4 avec un polysaccharide non ramifié ou un polyanion est couplé à une autre phase solide particulaire non cellulaire, de préférence à des particules de latex.
